(19) **Europäisches Patentamt European Patent Office Office européen des brevets**

(11) **EP 4 422 488 B1**

(12) **EUROPEAN PATENT SPECIFICATION**

(45) Date of publication and mention
of the grant of the patent:
**03.12.2025  Bulletin 2025/49**

(21) Application number: **22808696.3**

(22) Date of filing: **20.10.2022**

(51) International Patent Classification (IPC):
***A61B 5/113*** (2006.01)     ***A61B 5/00*** (2006.01)
***A61B 5/08*** (2006.01)

(52) Cooperative Patent Classification (CPC):
**A61B 5/1135; A61B 5/08; A61B 5/7239;
A61B 5/7278**

(86) International application number:
**PCT/EP2022/079222**

(87) International publication number:
**WO 2023/072727 (04.05.2023 Gazette 2023/18)**

(54) **SYSTEM AND METHOD FOR ANALYZING PHYSIOLOGIC SIGNALS**

SYSTEM UND VERFAHREN ZUR ANALYSE PHYSIOLOGISCHER SIGNALE

SYSTÈME ET PROCÉDÉ D'ANALYSE DE SIGNAUX PHYSIOLOGIQUES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **26.10.2021  US 202163271905 P
25.05.2022  EP 22175575**

(43) Date of publication of application:
**04.09.2024  Bulletin 2024/36**

(73) Proprietor: **BIOTRONIK SE & Co. KG
12359 Berlin (DE)**

(72) Inventors:
• **DE VOIR, Christopher S.
Portland, Oregon 97201 (US)**
• **GUHANIYOGI, Shayan
Portland, Oregon 97206 (US)**
• **WHITTINGTON, R. Hollis
Portland, Oregon 97202 (US)**
• **REDDY, Ravi Kira Kondama
Portland, Oregon 97221 (US)**

(74) Representative: **Biotronik Corporate Services SE
Corporate Intellectual Property
Sieversufer 7-9
12359 Berlin (DE)**

(56) References cited:
**US-A- 5 300 093       US-A1- 2016 279 361
US-A1- 2017 164 906**

**Description**

Embodiments of the present disclosure relate to a system for analyzing physiologic signals, a method for analyzing physiologic signals, and a computer-readable storage medium for executing the method. Embodiments of the present disclosure more particularly relate to a morphological analysis of one or more physiologic signals in an implantable medical device (IMD).

Implantable medical devices are widely used to monitor a patient's health status, e.g., by analyzing physiologic signals measured by implantable medical devices. Examples of physiologic signals which can be measured by implantable medical devices include, but are not limited to, ECG, SpO2, blood pressure, activity, fluid status, inspiration/expiration efforts, disordered breathing and respiration.

However, it can be difficult to obtain meaningful diagnostic information from the raw signals for a variety of reasons. For example, patients with monitoring-only devices may have more mobility/movement, which can interfere with measurements. In addition, there is usually no single time of day when all physiologic processes coincide, but rather a window or region of time.

Some devices for analyzing physiologic signals use conventional digital filters. However, conventional digital filters have limited out of band suppression characteristics and group delay, which leads to distortion of morphological features and ultimately to non-recognition or erroneous measurement of the morphological features. Other devices for analyzing physiologic signals use non-time domain methods (e.g. statistic and/or frequency domain), such that, once the signal is processed, the event-by-event information is lost, and relevant information can no longer be analyzed. Furthermore, such processing must aggregate measurements over time, so early signs of deterioration may be missed. Ultimately, an inaccurate estimate of the physiologic event results. Relevant prior art is disclosed in US5300093 and US2016/279361.

**In** light of the above, a system for analyzing physiologic signals, a method for analyzing physiologic signals, and a computer-readable storage medium for executing the method that overcomes at least some of the problems in the art are beneficial.

It is an object of the present disclosure to provide a system for analyzing physiologic signals, a method for analyzing physiologic signals, and a computer-readable storage medium for executing the method which can improve an analysis of physiologic signals. **In** particular, an object of the present disclosure is to accurately determine one or more physiologic parameters based on an analysis of physiologic signals.

The objects are solved by the features of the independent claims. Preferred embodiments are defined in the dependent claims.

According to an independent aspect of the present disclosure, a system for analyzing physiologic signals is provided. The device includes an input configured to receive a discrete-time physiologic signal; and at least one processor module. The at least one processor module is configured to:

- compare a sign associated with a present sample value of the physiologic signal to a sign associated with a previous sample value of the physiologic signal;
- determine a presence of a fiducial event if the sign associated with the present sample value is different from the sign associated with the previous sample value;count samples of the physiologic signal in intervals defined between fiducial events to obtain a respective sample count for each of the intervals; and
- determine at least one physiologic parameter based on the sample counts.

According to some embodiments which can be combined with other embodiments described herein, the at least one processor module is configured to:

- compare a sign associated with a present sample value of the physiologic signal to a sign associated with a previous sample value of the physiologic signal;
- determine a presence of a fiducial event if the sign associated with the present sample value is different from the sign associated with the previous sample value;
- determine the acceptability of the fiducial event for inclusion or exclusion in determining intervals or parameters from the state of flags indicating rejection criteria passed or failed over the analytical interval;
- count samples of the physiologic signal in intervals defined between fiducial events to obtain a respective sample count for each of the intervals; and
- determine at least one physiologic parameter based on the sample counts.

According to some embodiments, which can be combined with other embodiments described herein, the physiologic signal is selected from the group including, or consisting of, an electrocardiogram (ECG), oxygen saturation (SpO2), blood pressure, impedance, accelerometry, activity, fluid status, inspiration and/or expiration efforts, and respiration.

Preferably, the physiologic signal is a respiration. Respiration is a particular example of a meaningful physiologic signal that can indicate normal breathing function, or disorders like COPD, asthma, pulmonary edema, emphysema, sleep apnea, Cheyne-Stokes syndrome, and others.

The term "discrete-time physiologic signal" as used throughout the present disclosure refers to a physiologic signal which has values occurring at distinct, separate points in time. That is, time is viewed as a discrete variable. The discrete-time physiologic signal can be obtained by sampling from a continuous-time [continuous-valued] physiologic signal. The discrete-time physiologic signal obtained by sampling a sequence at uniformly spaced times ("sample value of the physiologic signal") has an associated sampling rate.

The sign associated with the physiologic signal indicates a property of being either positive (+) or negative (-) relative to some baseline.

The term "fiducial event" as used throughout the present disclosure refers to a morphological feature in the physiologic signal. In particular, a fiducial event is a distinctive shape, structure, or feature in the physiologic signal which can be identified as such and used for further analysis.

According to some embodiments, which can be combined with other embodiments described herein, the fiducial event can be a zero-crossing or an extremum (minimum or maximum) of the physiologic signal.

According to some embodiments, which can be combined with other embodiments described herein, the fiducial event includes, or is, a zero-crossing.

Preferably, the at least one processor module is configured to identify the zero-crossing of a baseline-centered signal if the sign associated with the present sample value differs from the sign associated with the previous sample value. For example, a zero-crossing can be identified when the sign switches from negative to positive or from positive to negative.

Preferably, the sample count of an interval between two successive zero-crossings corresponds to counts of an unbroken run of a positive sign (e.g., positive lobe values) or a negative sign (e.g., negative lobe values) of the sample values in the interval. Upon each zero-crossing, the counts of negative and positive lobes can be summed and stored as a duration in counts of a known sample rate.

Preferably, upon each zero-crossing, the sample count for the next lobe is reset and begins accumulating the sample count again. For example, while the signal is negative, the count of negative samples is accumulated, and then latched while the signal is positive.

According to some embodiments, which can be combined with other embodiments described herein, the fiducial event includes, or is, an extremum.

Preferably, the at least one processor module is configured to identify the extremum if the sign associated with a slope the present sample value differs from the sign associated with a slope of the previous sample value.

According to some embodiments, which can be combined with other embodiments described herein, the extremum is a (local) maximum or a (local) minimum. Preferably, the maximum is a peak. Additionally, or alternatively, the minimum is a trough.

Preferably, the intervals for the sample count are defined between two successive extrema of the same type, e.g., between two peaks or between two troughs.

Preferably, the sample count of an interval between two successive extrema corresponds to counts of an unbroken run of a positive slope from a trough to a peak (e.g., ascent) or a negative slope from a peak to a trough (e.g., descent) of the sample values in the interval. Upon each extrema, the counts of ascents and descent can be summed and stored as a duration in counts of a known sample rate.

Preferably, upon each extrema, the sample count for the next ascent or descent is reset and begins accumulating the sample count again. For example, while the signal is rising a trough, the count of ascent samples is accumulated, and then latched when the signal reaches a peak.

According to the present invention the at least one processor module is configured to reject one or more fiducial events if one or more rejection criteria are met, wherein the one or more rejection criteria relate to an operating limit of at least one signal processing element of the system (e.g., because the sensor input exceeds the undistorted operating limits of the quantizer).

In other words, the rejected fiducial event(s) is/are not used in the determining of an interval and/or the at least one physiologic parameter.

Preferably, further rejection criteria relate to at least one of a patient's motion (e.g., based on acceleration data provided by an acceleration sensor of an IMD), non-monotonicity (e.g. of an ascent or descent) and noise (e.g., a noise floor).

According to some embodiments, which can be combined with other embodiments described herein, the system is configured to determine the at least one physiologic parameter using sample counts associated with two or more fiducial events.

There may be more than one fiducial event. Preferably, the two or more fiducial events are of different types. It all depends on how the intervals are being measured to create one whole physiologic interval. If the interval spans fiducial events of the same type, then the count between them may span one whole physiologic interval. This would be the case if the configuration was set to, for example, only use positive going zero-crossings to measure the physiologic interval and the other fiducial intervals were set to disregard. If the fiducial events used to measure the interval are of opposite types, for example, zero-crossing up to zero-crossing down, the only ½ of the physiologic interval was measured and it would require to pair it with the last or next zero-crossing down to zero-crossing up to complete the physiologic cycle. Two half phases compose one whole physiologic cycle, for example, positive time + negative time, another example, ascent time + descent time.

In a preferred embodiment, the fiducial analysis is broken down even further into 4 phases because of signs and slopes. An example, trough to zero-crossing up to peak to zero-crossing down to trough. In this case it would be required to add up the 4 sub-intervals to yield the duration of 1 physiologic cycle. The more broken down whole physiologic cycles are, the measurements take place within a whole physiologic cycle and goes to the point of the x4 statistical leveraging mentioned later.

Preferably, the two or more fiducial events are selected from the group including, or consisting of, an upwards zero-crossing (zero-crossing Up), a downwards zero-crossing (zero-crossing Down), a peak, and a trough.

According to some embodiments, which can be combined with other embodiments described herein, the system is configured to determine the at least one physiologic parameter for one or more observation windows and/or as needed by the yield of accepted fiducials and the number of required fiducials to satisfy the resolution requirement that is dependent on the breath rate.

Preferably, a length of each observation window is based on a set minimum number of samples and/or a maximum processing time. In particular, an event count limit (lower bound) is provided whose value, in a preferred embodiment, is calculated from the required minimum number of samples to estimate the respiratory rate to a resolution of ±2Bpm with 95% confidence, for example, discriminating between 16Bpm and 18Bpm. Second, a time limit (upper bound) is provided to constrain the fiducial analysis from proceeding overlong because signal properties are not yielding sufficient accepted fiducial events to satisfy the former.

According to some embodiments, which can be combined with other embodiments described herein, the system includes an implantable medical device.

Preferably, the implantable medical device is configured to receive or generate the discrete-time physiologic signal. For example, the implantable medical device includes, or is connected to, one or more sensors configured to measure the discrete-time physiologic signal or a physiologic signal from which the discrete-time physiologic signal is derived.

Preferably, the implantable medical device is configured to perform at least the aspects of comparing signs, determining a presence of a fiducial event, and count samples.

In some embodiments, the implantable medical device can be configured to determine the at least one physiologic parameter based on the sample counts. In particular, the analysis can be implemented wholly in the implantable medical device. Alternatively, the implantable medical device could provide a fiducial analytical output to an external entity (e.g., a server or other device), and the calculation of physiologic parameters could be completed in whole or in part by some other means, such as the server or other device.

According to some embodiments, which can be combined with other embodiments described herein, the system is configured to transmit the determined at least one physiologic parameter to at least one external entity via a communication interface of the system, in particular a communication interface of the implantable medical device.

Preferably, the at least one external entity is a mobile terminal of a physician and/or a web (e.g. website and/or web interface) of the physician and/or a web service used by the physician, e.g. a remote server with a web interface facing the physician.

Preferably, the mobile terminal of the physician is selected from the group including (or consisting of) a smartphone, a personal digital assistant, a tablet, a notebook, a smart watch, any device with a web browser, and smart glasses.

Preferably, the communication interface is a wireless communication interface. However, the present disclosure is not limited thereto, and the communication interface can be a wired communication interface.

According to some embodiments, which can be combined with other embodiments described herein, the communication interface is configured for communication via at least one communications network, in particular the Internet.

Preferably, the at least one communications network includes, or is, a local area network and/or a wide area network.

Preferably, the wide area network is configured for at least one of Global System for Mobile Communications (GSM), General Package Radio Service (GPRS), Enhanced Data Rates for GSM Evolution (EDGE), Universal Mobile Telecommunications System (UMTS), Long-Term Evolution (LTE), and Fifth Generation Technology Standard (5G).

According to another independent aspect of the present disclosure, a method for analyzing physiologic signals is provided. The method includes:

- receiving a discrete-time physiologic signal;
- comparing a sign associated with a present sample value of the physiologic signal to a sign associated with a previous sample value of the physiologic signal;
- determining a presence of a fiducial event if the sign associated with the present sample value is different from the sign associated with the previous sample value;
- counting samples of the physiologic signal in intervals defined between fiducial events to obtain a respective sample count for each of the intervals; and
- determining at least one physiologic parameter based on the sample counts.

According to some embodiments, which can be combined with other embodiments described herein, the system and/or the method includes a control mechanism capable of selecting any subset of the four analytical fiducial events and enabling or disabling any subset of the four rejection criteria in the preferred embodiment.

Embodiments are also directed at systems/devices for carrying out the disclosed methods and include system/device aspects for performing each described method aspect. These method aspects may be performed by way of hardware components, a computer programmed by appropriate software, by any combination of the two or in any other manner. Furthermore, embodiments according to the invention are also directed at methods for operating the described device/system. The disclosure includes method aspects for carrying out every function of the device/system.

According to another independent aspect of the present disclosure, a machine-readable medium is provided. The machine-readable medium includes instructions executable by one or more processors to implement the method for analyzing physiologic signals of the embodiments of the present disclosure.

The (e.g., non-transitory) machine readable medium can include, for example, optical media such as CD-ROMs and digital video disks (DVDs), and semiconductor memory devices such as Electrically Programmable Read-Only Memory (EPROM), and Electrically Erasable Programmable Read-Only Memory (EEPROM). The machine-readable medium can be used to tangibly retain computer program instructions or code organized into one or more modules and written in any desired computer programming language. When executed by, for example, one or more processors such computer program code can implement one or more of the methods described herein.

According to another independent aspect of the present disclosure, a device or system for analyzing physiologic signals is provided. The device or system includes one or more processors; and a memory (e.g., the above machine-readable medium) coupled to the one or more processors and comprising instructions executable by the one or more processors to implement the method for analyzing physiologic signals of the embodiments of the present disclosure.

According to some embodiments, which can be combined with other embodiments described herein, the device and/or system comprises a means for analyzing physiologic signals that measures a whole physiologic period, or halves, or quarter phases, or some other fraction corresponding to chosen fiducial points such that there are multiple overlapping analysis intervals per whole physiologic period producing higher statistical power in the estimated parameter.

According to some embodiments, which can be combined with other embodiments described herein, the device and/or system comprises a means for analyzing physiologic signals that preserves the fidelity of a physiologic signal, evaluates that signal for departures from its expected shape, such as non-monotonicity, and sets an indicator if the signal departs from that expectation for each fraction of a whole period.

According to some embodiments, which can be combined with other embodiments described herein, the device and/or system comprises a means for analyzing physiologic signals that evaluates against configurable morphological or interference rejection criteria each whole, half, quarter, or fraction of a period selecting which overlapping analysis intervals are included or omitted from contributing to the estimated parameter.

According to some embodiments, which can be combined with other embodiments described herein, the device and/or system comprises a means for analyzing physiologic signals that evaluates against statistics or quality metrics each whole, half, quarter, or fraction of a period selecting which overlapping analysis intervals are included or omitted from contributing to the estimated parameter.

According to some embodiments, which can be combined with other embodiments described herein, the device and/or system comprises a means for analyzing physiologic signals prospectively, adaptively, or retrospectively selects a subset of at least one or a combination of at least two from multiple sensor inputs with the best statistical properties, or quality metrics for additional observation, calculating parameters, storing, or reporting results to a server.

According to some embodiments, which can be combined with other embodiments described herein, the device and/or system comprises a means for analyzing physiologic signals that preserves the fidelity of a physiologic signal, evaluates that signal for departures from its expected rate of change, such as a sigh, gasp, or other breathing anomaly, and sets an indicator if the signal departs from that expectation for each fraction of a whole period.

So that the manner in which the above recited features of the present disclosure can be understood in detail, a more particular description of the disclosure, briefly summarized above, may be had by reference to embodiments. The accompanying drawings relate to embodiments of the disclosure and are described in the following:

Fig. 1     shows an exemplary generation of zero-crossing fiducials according to embodiments of the present disclosure;

Fig. 2     shows an exemplary calculation of a zero-crossing duration according to embodiments of the present disclosure;

Fig. 3     shows a zero-crossing unit according to embodiments of the present disclosure;

Fig. 4     shows an exemplary generation of extrema fiducials according to embodiments of the present disclosure;

Fig. 5     shows an exemplary calculation of an extrema duration according to embodiments of the present disclosure;

Fig. 6     shows an extrema unit according to embodiments of the present disclosure;

Fig. 7     shows a storage and observation control unit according to embodiments of the present disclosure;

Fig. 8     shows fiducial events and their effective rates per minute;

Fig. 9     shows a fiducial control unit according to embodiments of the present disclosure; and

Fig. 10    shows the overlap between types of fiducial intervals.

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure. Further, features illustrated or described as part of one embodiment can be used on or in conjunction with other embodiments to yield yet a further embodiment. It is intended that the description includes such modifications and variations.

Implantable medical devices are widely used to monitor a patient's health status, e.g., by analyzing physiologic signals measured by the implantable medical devices. However, it can be difficult to obtain meaningful diagnostic information from the raw signals for at least some of the following reasons:

- Patients with monitoring-only devices may have more mobility/movement, which can interfere with measurements.
- There is usually no single time of day when all physiologic processes coincide.
- The optimal subset of sensors needed for a diagnosis or for tracking of a health status varies with the implant location and the patient's position of rest.
- There are morphological features in physiologic data that are analogous to a false summit, maximum, or minimum.
- There are device inputs that are noise or interference that falsely appear as or obscure actual morphological features.
- Intermittent respiratory sighs have larger volume which is expressed as higher amplitude in sensor output that can exceed the undistorted operating limits of some signal processing chains. Here the point may be that the AutoGain function must leave some operating margin and/or guard banding for expected physiologic changes in amplitude so the signal level remains with the undistorted operating limits of the signal processing path. AutoGain improves a discrete gain function by updating gains locally per speed range. It segments a movement into submovements to update a profile of aiming errors, which it tries to reduce by adapting the gain response.
- Heart failure may be indicated by as little as a two breath per minute (Bpm) changes from baseline and is thus hard to detect.

Additionally, increased respiratory sighs or changes in the duration of the sighs (maxima's of the amplitude variance) may be ways to determine the change in the physiological state of the individual.

The embodiments of the present disclosure provide an improved morphological analysis of physiologic signals. In particular, the embodiments of the present disclosure analyze the morphology of physiologic signals available through sensors in IMDs to yield information about the patient's health state, especially properties of respiration. In particular, signs of a discrete-time physiologic signal are analyzed to determine fiducial events, and the fiducial events are in turn used to determine a physiologic parameter indicative of a patient's health status.

In detail, a system for analyzing physiologic signals according to the present disclosure includes an input configured to receive a discrete-time physiologic signal; and at least one processor module. The physiologic signal can be an electrocardiogram (ECG), oxygen saturation (SpO2), blood pressure, impedance, accelerometry, activity, fluid status, inspiration and/or expiration efforts, or respiration. Preferably, the physiologic signal is a respiration.

The at least one processor module is configured to compare a sign (positive or negative) associated with a present sample value of the physiologic signal (e.g., respiration) to a sign associated with a previous sample value of the physiologic signal; determine a presence of a fiducial event (e.g., zero-crossings or extrema) if the sign associated with the present sample value is different from the sign associated with the previous sample value; count samples of the physiologic signal in intervals defined between fiducial events to obtain a respective sample count for each of the intervals (the intervals may be 1 whole, 2 halves, or 4 phases of a whole physiologic cycle); and determine at least one physiologic parameter (e.g., respiration efforts, respiration distress and/or interruptions in respiration) based on the sample counts.

Accordingly, signs of the discrete-time physiologic signal are analyzed to determine fiducial events, and the fiducial events are in turn used to determine a physiologic parameter indicative of a patient's health status. For example, the device for analyzing physiologic signals may accept intracardiac electrograms (IEGMs), serial electrocardiograms (SECGs), impedance, accelerometer data, and/or pressure input streams, quantize them, pre-condition the inputs in a non-distorting manner, select observations times (especially those that reveal heart failure), optimize a subset of inputs, extract analytical fiducial events and the relationship between them, handle interfering or confounding factors, produce an output including statistics and quality metrics, and upload the results to a server.

Thereby, a meaningful measurement of a physiologic parameter, such as respiration, respiration efforts, respiration distress and interruptions in respiration, that can be used to monitor, track, or diagnose the occurrence of a disease, such as heart failure (HF), can be provided.

The following is an overview of a process for determining physiologic parameters, wherein the process implements the fiducial event analysis of the embodiments of the present disclosure.

Overview:

    A. Synchronizing observations to a time or a physiologic parameter
    B. Search of sensor offset and gain settings
    C. Signal pre-conditioning to remove residual offset and out of band signal content
    D. Fiducial algorithm yielding interval data, statistics, and quality metrics
    E. Prospective selection from among multiple sensors using statistics and quality metrics.
    F. Determination of physiologic parameters from accepted interval data

Details:

A. Synchronizing to observations to a time or a physiologic parameter

Embodiments of the present disclosure can determine the expected daily cardiopulmonary, metabolic, or autonomic nadir to sample the input based on time of day, heart rate, heart rate variance, indicator of autonomic tone, temperature, movement, posture, or pressure or subsets of these to indicate an optimal time for measurement. Thus, a measurement may be triggered by a combination of these, or is on standby or suspended until a subset of these subsides.

B. Search of sensor offset and gain settings

Embodiments of the present disclosure can determine or adjust in real-time configurable parameters of the sensor unit, such as DC offset and gain, by an iterative means, such as Fibonacci Search, so that that the sensor output signal level remains within undistorted operating limits. Further, the output level can be guard-banded if fixed or adaptive so that the signal level remains so despite position, movement, or physiologic variations, such as spontaneous augmented breaths (sighs).

C. Signal pre-conditioning to remove residual offset and out of band signal content

Embodiments of the present disclosure can pre-condition the input to approximate zero mean and reduce the out of band signal content by a means, such as a Wavelet Filter that preserves the fidelity of morphology of interest so that the actual time relationship between fiducials can be determined including but not limited to times and slopes of zero-crossings, times and amplitudes of peaks and troughs, areas under lobes and rising and falling partitions of lobes, areas and proportions of these.

D. Fiducial algorithm yielding interval data, statistics, and quality metrics

a) The embodiments of the present disclosure can accept the pre-conditioned input and evaluate fiducial events intervals, amplitudes, slopes and extrema, associating each with a time index, sample value, and duration. This concept is described later in more detail.

b) The embodiments of the present disclosure can evaluate the pre-conditioned input and associate each fiducial event state against a set of rejection criteria to tag each with a flag that indicates noise floor, non-monotonic signal changes, rail or motion flag. This concept is described later in more detail.

c) The embodiments of the present disclosure can subdivide one whole physiologic interval so that the analytic subintervals overlap the whole interval multiple times increasing the power of the estimate. For examples, a single physiologic cycle, such as a breath interval or cardiac interval N-times, where N number of distinct fiducials analyzed, for example, each breath interval can be oversampled by 4 analytical intervals: two Zero-Crossing intervals (one zero-crossing up, one zero crossing down), two Extrema intervals (one Peak-to-peak, one Trough-to-trough) and thus reduce the uncertainty around the estimate by half.

E. Prospective selection from multiple sensors

Embodiments of the present disclosure can accept the pre-conditioned input and prospectively determine which of a subset of sensors either alone or combined best represents the physiologic signal of interest employing a combination of fiducials, rejection criteria, or quality metrics.

F. Determination of physiologic parameter

a) The embodiments of the present disclosure can select which fiducials or rejection criteria to include or exclude based on a set of predefined settings or adaptively changes the selection with changing signal properties.

b) The embodiments of the present disclosure can collect observations from those fiducials and rejection criteria selected by the configuration settings and provide instantaneous values or estimates from aggregates.

c) The embodiments of the present disclosure can suspend or halt a measurement in progress when confounding signals arise or an excessive number of rejection criteria are detected whereupon the feature is either recalibrated and/or the measurement is cancelled, suspended and resumed when the interfering signal subsides, or rescheduled.

d) The embodiments of the present disclosure can provide estimates of central tendency, variation, and confidence limits for the measurements on the accepted observations.

e) The embodiments of the present disclosure can determine metrics of quality for the collected data, such as the proportion of accepted to total observations.

f) The embodiments of the present disclosure can determine if the resolution of the measurement is sufficient, for example, answers the question "were required number of observations N obtained and accepted (and/or evaluated) that a breath rate of $X \pm Y$ (Bpm) can be reported with 95% confidence and a quality metric".

The morphological analysis described above in steps A-F may account for at least some of the following aspects:

- Preservation of a fidelity of signal morphology so that the actual time relationship between signal features can be determined.
- Compensation for patient movement or position or physiologic change so that the signal continues to contain physiologically significant and comparable information.
- Incorporation of multiple cues of the daily nadir (minimum) of sympathetic activity.
- Selection from the available sensors to provide coverage during the measurement time for a given posture.
- Analysis of multiple fiducials for each event to provide statistical power for the analysis.
- Customization of retention and rejection of analytical events to ensure robustness of results.
- Analysis of single events in multiple ways to increase the statistical power of the estimator.
- Customization of which analytical events are omitted or retained to increase efficiency.
- Conservation of device expenditure: suspend, standby, reschedule, or skip analysis when confounders arise.
- Instantaneous or collected observations.

- Provision of statistical estimates of calculated parameters.
- Provision of metrics for measurement quality and resolution.

The fiducial analysis of step D is described in more detail below.

According to some embodiments, which can be combined with other embodiments described herein, the fiducial event can be a zero-crossing or an extremum (minimum or maximum) of the physiologic signal.

## 1. Zero-crossing fiducial events

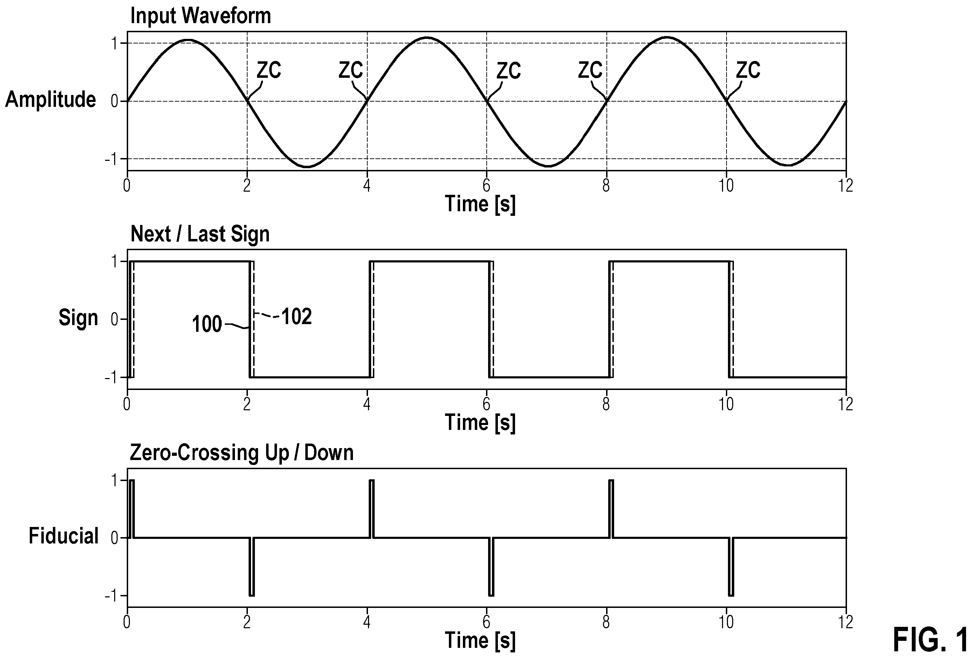

The steps that yield the zero-crossing fiducial events for analysis are demonstrated in FIG. 1. In the upper subplot, an example waveform is shown whose amplitude is within the undistorted operating range of the signal path versus time scale of seconds. The middle subplot shows the sign of each input sample, where a first line 100 corresponds to the sign of the present sample, and a second line 102 corresponds to the sign of the prior sample. The lower subplot shows the identification of the zero-crossing fiducial events ZC when the present and prior signs differ, where +1 is Up and -1 is Down, respectively.

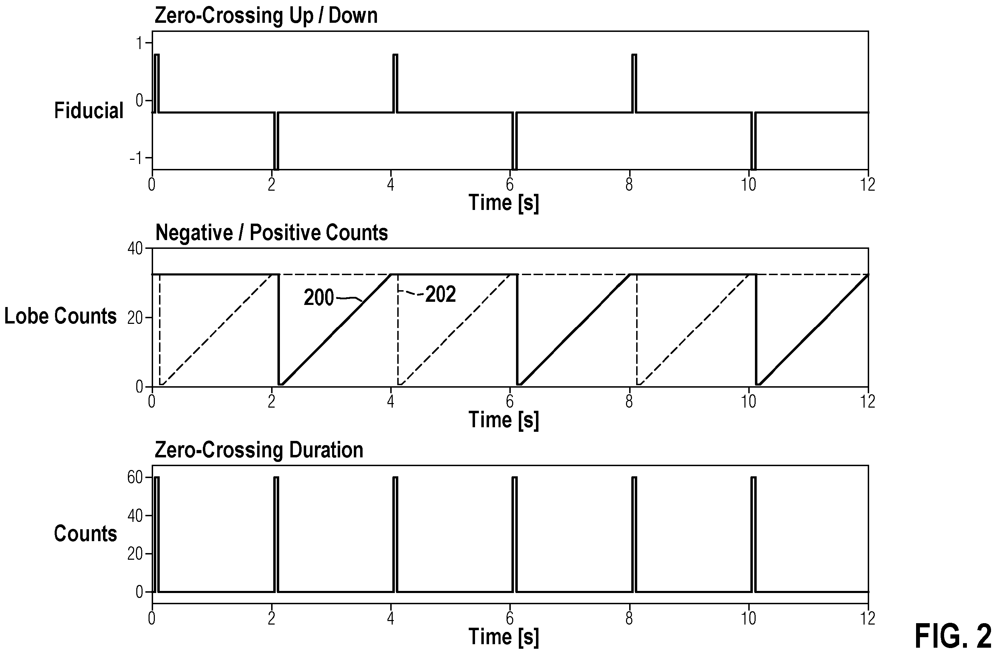

The zero-crossing fiducial event interval duration is calculated on each zero-crossing identified (upper subplot in FIG. 2). The middle subplot shows that while the sign of the signal remains the same, the number of samples are counted, where a first line 200 corresponds to counts of an unbroken run of negative lobe values and a second line 202 counts of positive lobe values. Upon each zero-crossing, the counts of negative and positive lobes are summed and stored as a duration in counts of a known sample rate (lower subplot).

Upon each zero-crossing, the sample count for the next lobe is reset and begins accumulating the sample count again. For example, while the signal was negative between 2s and 4s, the count of negative samples is accumulated, and then latched while the signal is positive between 4s and 6s, summed with the positive sample count at the zero-crossing Down (-1) at 6s, and restarted counting negative samples again. For this example, the positive sample count is latched at 2s as the signal turns negative, summed with the negative sample count at 4s, restarted, and latched at 6s as the signal turns negative.

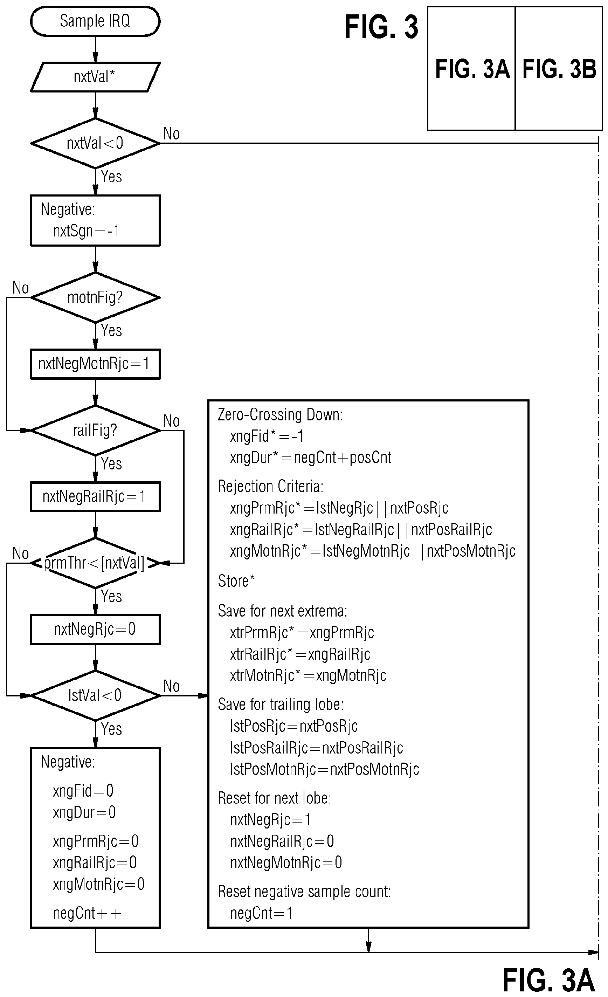

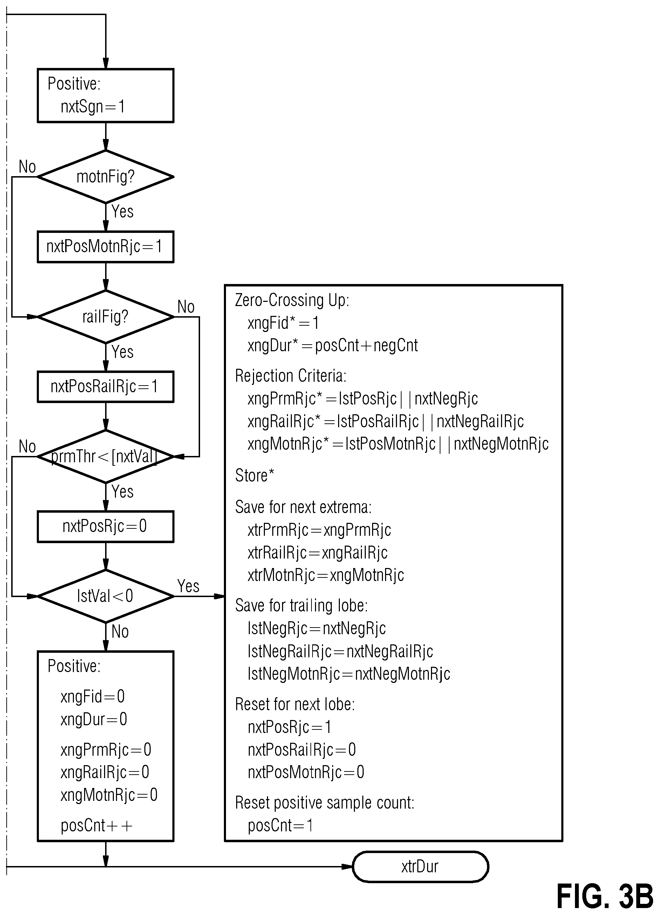

As shown in FIG. 3, in an exemplary embodiment, the above is accomplished by the following means.

Sample IRQ:

In an embodiment, the workflow enters the zero-crossing unit (FIG. 3) in real time on a sample-by-sample basis. The system has analog sensors that are sampled yielding a discrete time discrete valued data stream. The system issues an interrupt when a sample is available (Sample IRQ).

nxtVal:

The sample value (nxtVal*) is retrieved from the quantizer output register. Variables marked with an asterisk will later be saved at a step Store*. Test of whether nxtVal is negative is the entry-point for the zero-crossing unit (FIG. 1). The Xing unit must evaluate a negative lobe (left branch) and a Positive lobe (right branch).

nxtSgn:

The sign of nxtVal is assigned as positive or negative (nxtSgn).

motnFlg:

The system has an accelerometer and a unit to generate a motion flag which is sampled and its state is set if motion is active (nxtNegMotnRjc / nxtPosMotnRjc).

railFlg:

Because the sensor input may exceed the undistorted operating limits of the quantizer, the rail flag is sampled and its state is latched if the signal input is reaching the operating limits (nxtNegRailRjc / nxtPosRailRjc).

prmThr:

If the amplitude of negative and positive lobes exceeds the noise floor (prmThr) the prominence rejection flag for the respective lobe is cleared nxtNegRjc / nxtPosRjc, otherwise it is left in the true state. In other words, amplitude is assumed to be indistinguishable from noise until its amplitude demonstrates otherwise.

IstVal:

If the workflow is now in a negative or positive nxtVal branch, the sign of the last value (IstVal) is recalled to determine whether the present sample is crossing zero.

Negative / positive:

In the case that both the prior (IstVal) and the present (nxtVal) have the same sign, the fiducial identifier (xngFid), the duration of the analytical interval (xngDur), prominence, rail, and motion rejection flags are cleared (xngPrmRjc, xngRailRjc, and xngMotnRjc). If the workflow is in the negative branch, the count of negative samples is maintained. Otherwise, if the workflow is in the positive branch, the count of positive samples is incremented.

Zero-crossing Down / Up:

In the case that the prior (IstVal) and the present (nxtVal) sample values have different signs, a zero-crossing has been identified. Upon a zero-crossing event, the following steps can be performed: The fiducial identifier is assigned (xngFid*) and the duration (xngDur*) of this analytical interval (last zero-crossing of the same direction to the present zero-crossing) is calculated.

Rejection criteria:

The rejection criteria xngPrmRjc*, xngRailRjc*, and xngMotnRjc* are tested and are set to true if a flag for either the negative or positive was set for the respective duration rejection criterion. At this point, a store operation is performed for any variables marked with an asterisk up to this point, where the store operation includes buffering in an array or aggregating in an accumulator. Store operations may further be filtered to omit any data that has any subset of the possible fiducial identifiers, durations deemed invalid, or any subset of rejection flags.

Store*:

Variables are stored at this point.

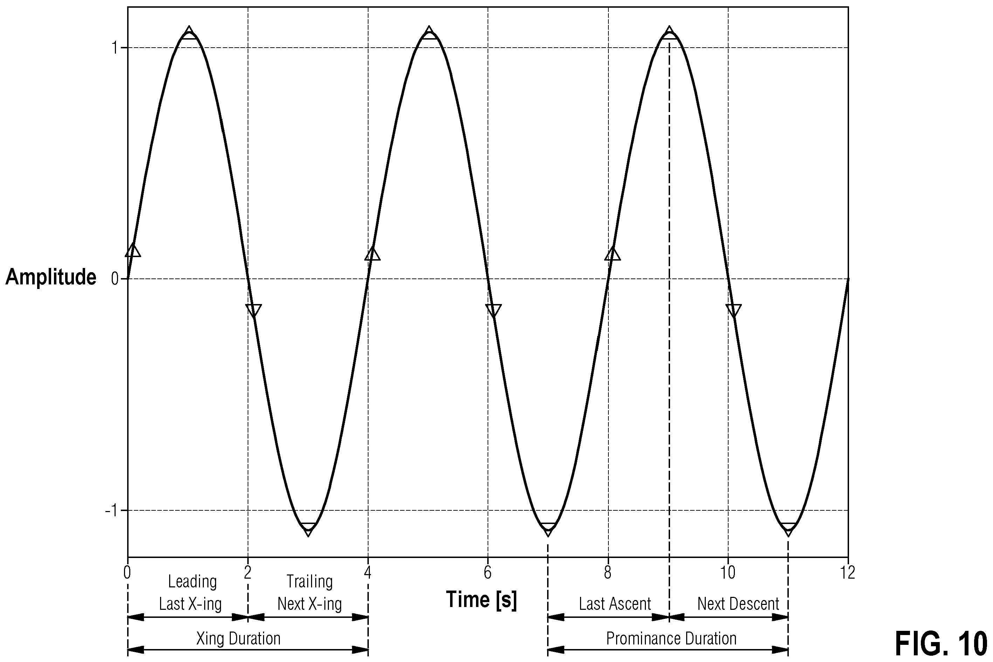

Fig. 10 shows that after an analysis interval is complete the associated breath duration, fiducial sample values, other status data is stored. When evaluating respiration as half of a whole physiologic interval, Leading (Next) data and stored as (Last) Trailing data. So, for example, if a rejection flag occurred between 7s and 9s and Prominence Duration was stored at 9s, the rejection data is carried over in the corresponding (Last) Trailing data, because the it lies within the span of Prominence Duration saved at 11s. After this, rejection flags for the Trailing Prominence data is allowed to expire. Similarly for Zero-Xing Duration.

Save for extrema:

The upcoming extrema event follows and partially overlaps the present zero-crossing analytical interval. Therefore, the analytical rejection states for noise floor (xngPrmRjc), rail (xngRailRjc), and motion (xngMotnRjc) are preserved for the upcoming extrema in xtrPrmRjc, xtrRailRjc, and xtrMotnRjc, respectively.

Save for trailing lobe:

A zero-crossing is the ending of a lobe and preserves the status of rejections flags for the trailing lobe for the upcoming analytical event. Present noise floor rejection flags (nxtPosRjc / nxtNegRjc), rail flags (nxtNegRailRjc / nxtPosRailRjc), and motion rejection flags (nxtNegMotnRjc / nxtPosMotnRjc) are preserved in their trailing counterparts (IstPosRjc / IstNegRjc), (IstNegRailRjc / IstPosRailRjc), and (IstNegMotnRjc / IstPosMotnRjc), respectively.

Reset for next lobe:

A zero-crossing is the beginning of a lobe and sets the noise floor rejection flag (nxtNegRjc / nxtPosRjc) to assume true until the signal magnitude exceeds threshold, and clears the rail (nxtPosRailRjc / nxtNegRailRjc) and motion rejection (nxtPosMotnRjc / nxtNegMotnRjc) flags.

Reset (negative / positive) sample count:

A zero-crossing is the beginning of a lobe and restarts the count of samples in the negative (negCnt) and positive (posCnt) lobes.

## 2. Extrema fiducial events

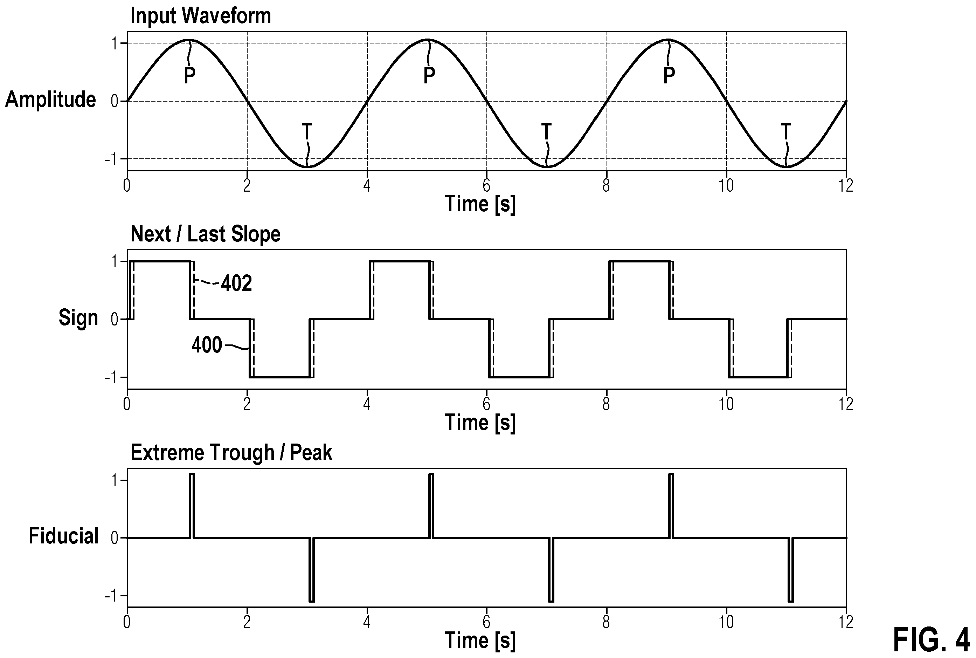

The steps that yield the extrema fiducial events for analysis are demonstrated in FIG 4. In the upper subplot, an example waveform is shown whose amplitude is within the undistorted operating range of the signal path versus time scale of seconds. The middle subplot shows the slope of each input sample up to the extrema, where a first line 400 corresponds to the slope of the present sample, and a second line 402 corresponds to the slope of the prior sample. The lower subplot shows the identification of the extrema fiducial events when the present and prior slopes differ at signal extrema, where +1 is Peak and -1 is Trough, respectively.

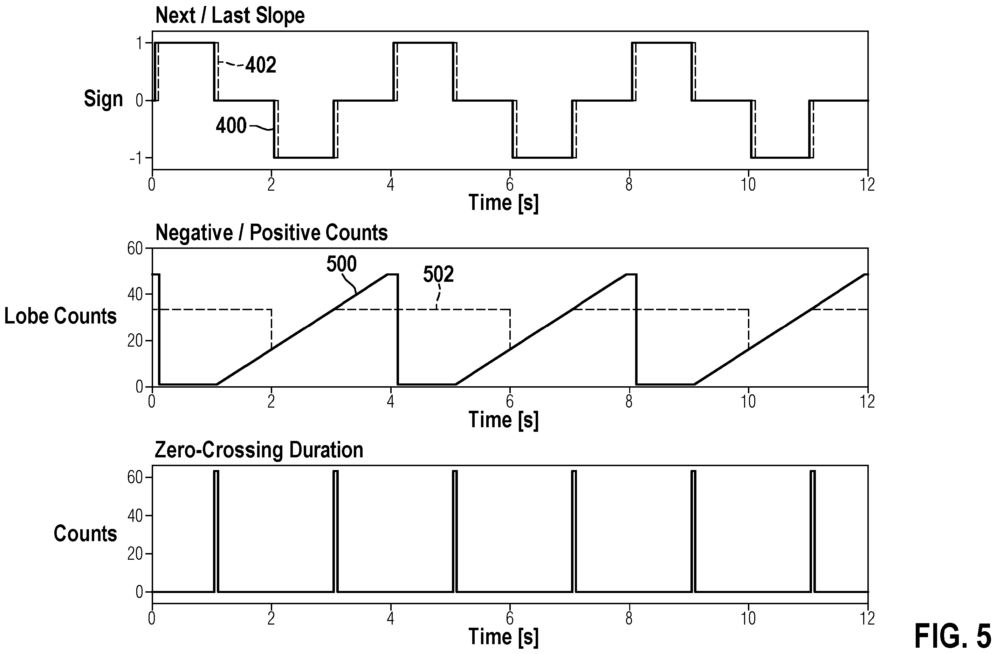

The extrema fiducial event interval duration is calculated on each extrema identified. The upper subplot of FIG. 5 shows the way points for latching and holding the duration of the descent from peak P to trough T. The middle subplot shows the free-running (reference numeral 500) and latched (reference numeral 502) descent counters. For example, as the signal comes off the peak P (sign of present (reference numeral 500) and previous (reference numeral 502) slopes differ at 5s in the upper subplot), the running descent counter starts (reference numeral 500 in the middle subplot). When the signal crosses zero (6s), the latched descent counter tracks the free funning counter. The latched descent counter stops tracking the free running counter upon reaching the nadir of the signal (7s). Upon reaching the trough T, the previous ascent (trough to peak) count and the present descent (peak to trough) count are summed yielding the trough to trough duration.

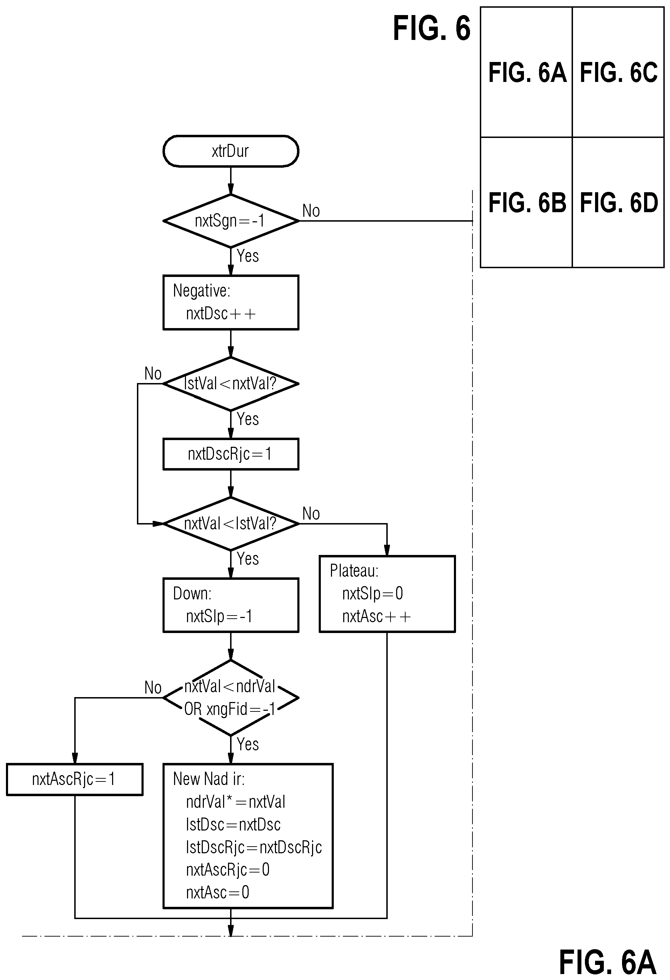

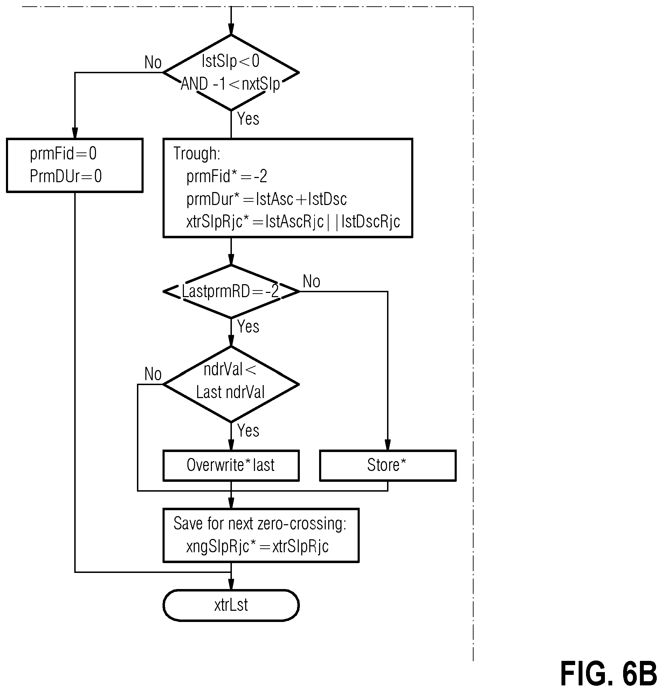

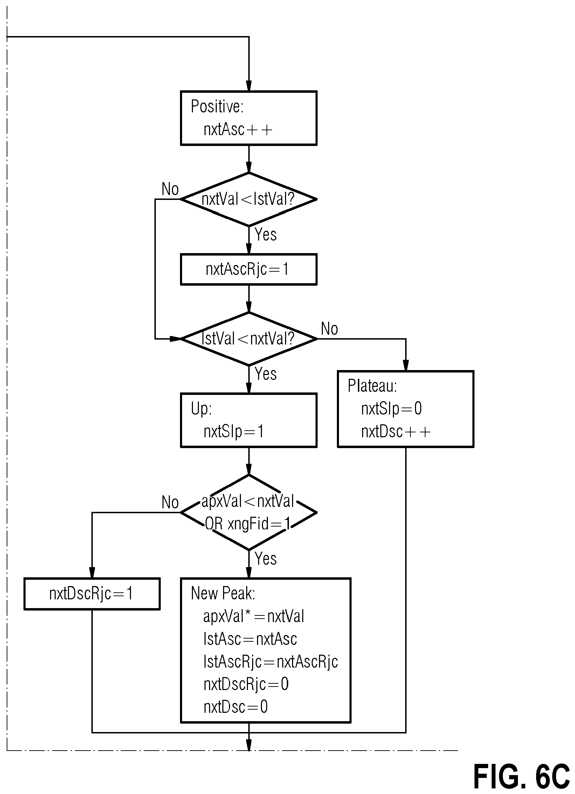

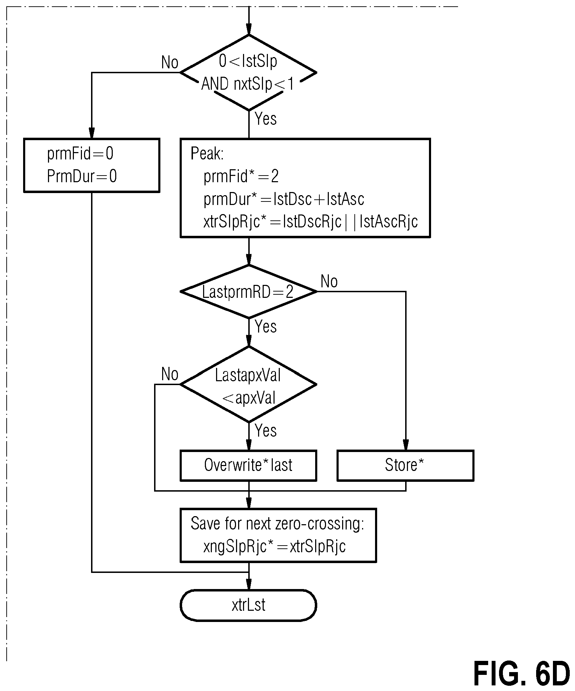

As shown in FIG. 6, in an exemplary embodiment, the above is accomplished by the following means.

xtrDur:

Upon completion of a sample through the zero-crossing unit, the workflow enters the slope unit (FIG. 6).

nxtSgn:

The workflow branches on whether nxtSgn indicates that the lobe is negative or positive.

Lobe negative / positive:

In the case that the lobe is negative, the count of samples of the slope descent is maintained. Otherwise, if the lobe is positive, the count of the slope ascent is incremented.

Non-monotonic rejection (post zero-crossing phase):

If the workflow is in the descent path, and a positive slope occurs, the flag to reject the descent (nxtDscRjc) will be latched. Encountering a negative slope on the ascent path will latch the ascent rejection flag (nxtAscRjc). Alternatively, the non-monotonic amplitude may be tested against a threshold to tolerate a non-zero noise floor. Also, alternatively, the rejection selector may be configured or adapted to ignore non-monotonicity. Note that a zero slope is a plateau and is not considered non-monotonic.

Plateau:

If the workflow is in the negative lobe branch and the slope is not negative, the workflow proceeds in the plateau path and skips seeking a deeper nadir, clearing the nxtSlp identifier. The ascent count is incremented because this path is also taken by the ascending limb after the deepest nadir for this analytical interval. If the workflow is in the positive lobe branch and the slope is not positive, the workflow proceeds in the plateau path and skips seeking a higher apex, clearing the nxtSlp identifier. The descent count is incremented because this path is also taken by the descending limb after the highest peak for this analytical interval.

Slope negative / positive:

The slope (nxtSlp) is determined to be negative or positive by testing the relationship between lstVal and nxtVal. If the present sample is also a zero-crossing (xngFid) that agrees with the slope or the present sample is a deeper nadir or high apex, the present value is stored as a possible extremum for this analytical interval. At a rapid physiologic rate that approaches the upper band limit, the signal value may represent an extrema for only a single sample. The present sample count of the descent or ascent limb (IstRjc / IstRjc) is cached and the rejection status (nxtDscRjc / nxtAscRjc) for non-monotonic changes in that limb is cached (IstDscRjc / IstAscRjc). The sample count and non-monotonic rejection for the upcoming ascent or descent limb is cleared by a deepening nadir and a higher apex because the corresponding new limb commences upon reaching a nadir or apex.

Non-monotonic Rejection (post extrema phase):

If the present sample follows an extremum (nadir / apex) and the sign of the lobe (nextSgn) and the slope agree, the non-monotonic rejection flag will be latched in the corresponding limb (nxtAscRjc /nxtDscRjc). Note that an extremum of greater magnitude may clear this flag.

Extremum trough / peak:

On each sample, the relationship between the prior and the present slopes (IstSlp / nxtSlp) is tested to determine if an extremum has occurred. Note that the nxtSlp may also be zero allowing for an extremum with a plateau instead of a single sample vertex. Upon identification, the prominence is identified as a trough or peak (prmFid). At this point, the time span of the prior and present slope limbs is calculated (prmDur) as it constitutes one of two analytical intervals between extrema, those fiducials being trough-ascent limb-descent limb-trough or peak-descent limb-ascent limb-peak. The rejection criteria of the ascent and descent limbs are tested and associated with the present analytical interval (xtrSlpRjc).

Extrema of greater magnitude:

If the present extremum (prmFid) and the previous extremum (last stored prmFid) are the same, the extreme values they achieved (present and stored ndrVal / apxVal) are compared to see if the present extremum has a greater magnitude. The present value overwrites or replaces the last if the magnitude is greater, otherwise it is discarded or discounted. If (the sign of) the present extremum (prmFid) and the previous extremum (last stored prmFid) differ, then the present value is stored because it is the first. In an alternative embodiment, in the case that the morphology is yet monotonic, the prmFid value is incremented upon each extremum of greater magnitude as a metric to characterize the smoothness of descents and ascents.

xngSlpRjc:

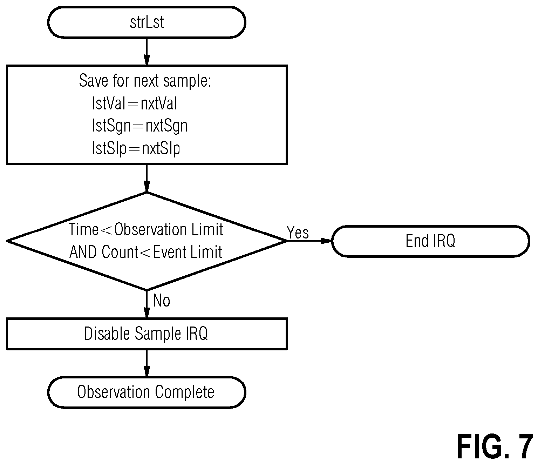

This value for xtrSlpRjec is cached for the upcoming zero-crossing.
Having described the Zero-Xing, Extrema, and Multiple Analytical Intervals in 1 whole Physiologic Interval of the fiducial algorithm (Step D), now the Storage, Computation, and Control aspects (Steps E and F) are described in more detail below with reference to FIGs. 7, 8, and 9.

strLst:

The above tests rely on the relationship between results achieved on the present and prior sample, therefore, nxtVal, nxtSgn, and nxtSlp are stored for the upcoming analytical event in IstVal, IstSgn, and IstSlp, respectively by the storage unit (FIG. 7).

Stored fiducial analysis events:

In a preferred embodiment, Store* results in caching four types of fiducial analysis events and their corresponding information collected: two types zero-crossings (Up and Down), and two types extrema (peak and trough). The associated data for each fiducial event are a sample number (sx), a fiducial value identifying type and direction (xngFid / prmFid), the greatest magnitude that occurred during the fiducial interval (nxtVal / ndrVal /apxVal), the duration of the previous and present fiducial intervals (xngDur / prmDur), and the zero-crossings and extrema interval rejection flags for the noise floor (PrmRjc), non-monotonicity (SlpRjc), signal level touching the clamp limit (RailRjc), and motion flag (MotnRjc).

**Table 1: stored fiducial analysis events**

| Xing Down | | | | | | | |
|---|---|---|---|---|---|---|---|
| SX | xngFid | ndrVal | xngDur | xngPrmRjc | xngSl pRjc | xngRailRjc | xngMotnRjc |
| | | | | | | | |
| Xing Up | | | | | | | |
| SX | xngFid | nxtVal | xngDur | xnPrmRjc | xngSl pRjc | xngRailRjc | xngMotnRjc |
| | | | | | | | |
| Trough | | | | | | | |
| SX | prmFid | ndrVal | prmDur | xtrPrmRjc | xtrSlpRjc | xtrRailRjc | xtrMotnRjc |
| | | | | | | | |
| Peak | | | | | | | |
| SX | prmFid | apxVal | prmDur | xtrPrmRjc | xtrSlpRjc | xtrRailRjc | xtrMotnRjc |

Observation Limit:

Upon conclusion of the sample IRQ, it can be tested whether the observation window is concluded either by exceeding a time limit or the count of analytical fiducial events is sufficient to calculate post-observation, parameters, statistics, and/or quality metrics. If the observation window is not complete, execution returns to the system. Otherwise, the sample IRQ is disabled, and the program flow proceeds to post-observation steps (FIG. 7).

Start:

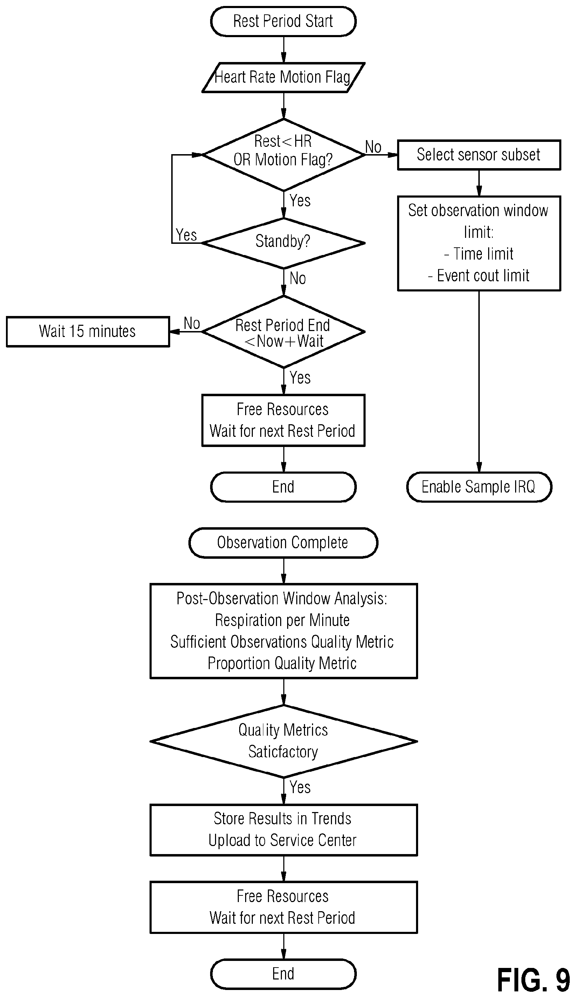

In a preferred embodiment, the workflow is started in the fiducial control unit on a time of day IRQ that indicates the beginning of an expected rest period (left hand side in FIG. 9). Alternatively, some physiologic state or external trigger (patient, clinician, auditory disordered breathing detection) may activate the start.

Interfering Conditions:

It is then checked that the actual heart rate does not exceed the rest limit. The motion flag is also checked to assure that transient physical activity is absent as it may result in a delayed chronotropic response falling in the upcoming observation window. In an alternative embodiment, temperature may also be used to indicate the daily metabolic nadir.

Standby:

In the case that the data do not satisfy the rest criteria, and standby is an enabled response, it is continued to monitor gating inputs as they refresh and resume the workflow when they cease.

Wait:

In the case that a standby is not an enabled response, and to reduce energy consumption of the IMD, it can be checked if a wait for a configurable amount of time (e.g., 15min) would falls outside the end of the rest period. If so, the fiducial control ceases until the next scheduled rest period start. Otherwise, the fiducial control resumes monitoring refresh of gating inputs after a wait. In the latter case, several attempts can be made to measure the physiologic signal during the rest period.

Select:

If the workflow passes the gate of interfering conditions, the sensor(s) for input can be selected. The sensor may be configurable to fixed, such as, a single preferred axis of a 3-axis accelerometer. Alternatively, the selection may be a prospective assessment of which of three accelerometer axes give the largest magnitude physiologic signal, such as tidal amplitude of breathing in the signal. Further alternatively, multiple signals may be arithmetically combined to for a single synthesized waveform for analysis. Still further alternatively, multiple signals from different sensor types may be multiplexed and their separate results used to corroborate, condition or improve the estimate of a calculated value, such as breathing rate.

Observation window limits:

There can be two observation window limits. First, an event count limit (lower bound) is provided whose value, in a preferred embodiment, is calculated from the required minimum number of samples to estimate the respiratory rate to a resolution of ±XBpm (e.g. ± 2Bpm) with YY% (e.g. 95 %) confidence, for example, discriminating between 16Bpm and 18Bpm. Second, a time limit (upper bound) is provided to constrain the fiducial analysis from proceeding overlong because signal properties are not yielding sufficient accepted fiducial events to satisfy the former. Thus, the observation window may complete if a sufficient number of accepted events occur before the time expires, lowering the device energy consumption. Alternatively, a parameter has been sequentially or incrementally calculated and is partially or completely available for storages or reporting upon observation complete. For example, analytical fiducial intervals may demonstrate transient effects outside the expect rate of change, such as sighs, gasps, or other breathing anomalies or disordered breathing patterns. In this case, an indicator would be set that the signal departs from such expectations.

Enable sample IRQ:

This activates the sample-by-sample processing of the signal stream in the zero-crossing unit (FIG. 3).

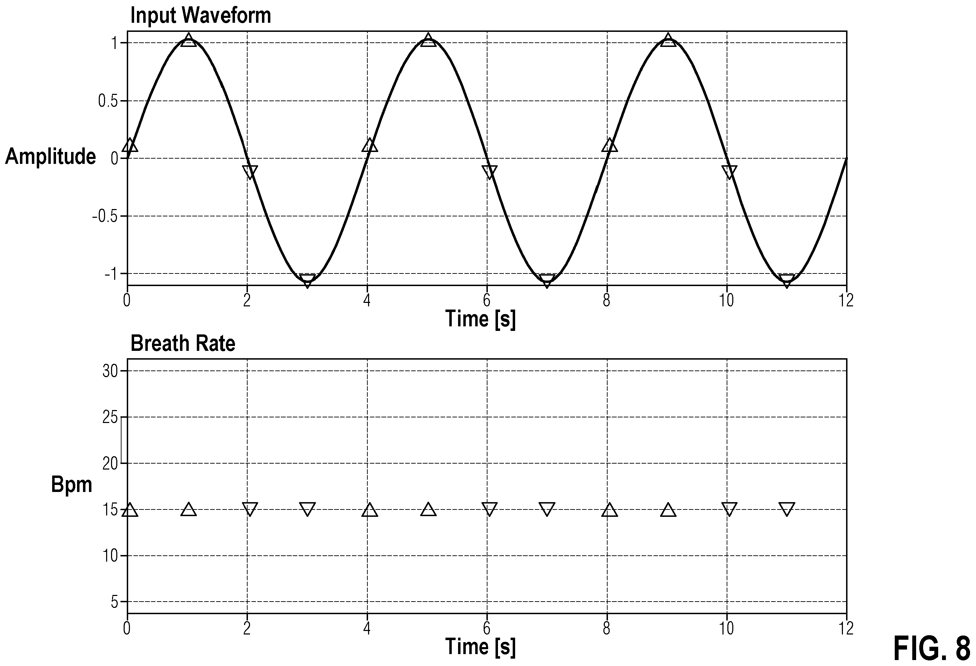

Now the determination of physiologic parameters of step G is described in more detail below with reference to FIGs. 8 and 9.

Observation complete:

The sample-by-sample analysis of fiducial events concludes with observation complete (FIG. 7) and the workflow passes to post-observation window analysis (right hand side in FIG. 9). The post-observation window analysis is performed on the stored fiducial analysis events of the entire observation window.

Rate per minute for an observation window:

The count of accepted and count of rejected analysis events that were configured to be included for analysis are counted. In the case the sensor was being used to measure breath rate, the durations in samples are summed and then normalized to allow conversion to a rate per minute (FIG. 8).

$$ax = i \mid [ZeroXingUp_i; ZeroXingDown_i; Apex_i; Nadir_i] \in Accepted$$

$$rx = i \mid [ZeroXingUp_i, ZeroXingDown_i, Apex_i, Nadir_i) \in Rejected$$

$$count_{accepted} = count(ax)$$

$$count_{rejected} = count(rx)$$

$$duration_{sum} = \sum Intervals_{ax}$$

$$duration_{normalized} = duration_{sum}/count_{accepted}$$

$$rate_{breath} = 60/\left(duration_{normalized}/Frequency_{sampling}\right)$$

Alternatively, the breath rate may be calculated by inferring the breathing frequency from the amplitude of the extrema, and the slope of the breath feature at the zero-crossing as:

$$S_0 = (y_2 - y_1)/T_s \, , where \; S_0 \; is \; the \; slope \; at \; the \; zero$$
$$- crossing, and \; T_s \; is \; the \; sample \; rate$$

$$S_0 = A_0 \cdot 2 \cdot \pi \cdot f_0,$$

where $A_0$ is the breath amplitude, and $f_0$ is the breathing frequency

$$given \; T_0 = 1/(2 \cdot \pi \cdot f_0)$$

$$rate_{breath} = 60/((A_0 \cdot 2 \cdot \pi)/S_0)$$

Sufficient observations quality metric for an observation window:

The sufficient observations quality metric is the required number of observations in a sample to be able to discriminate between two breath rates with a resolution of $\pm$2Bpm with 95% confidence in a preferred embodiment. In an alternative embodiment, the resolution is $\pm$1Bpm with 90% confidence. Further, the quality metric of sufficient observations is constrained to a value within [0% ... 100%].

$$count_{required} = \mathrm{ceil}\left(8/\left(\left((60/(rate_{breath} + 2Bpm)) - (60/rate_{breath})\right)^2\right)\right)$$

$$count_{required} = \max\left(count_{required}, 1\right)$$

$$qual_{sufficient} = \min\left(1, count_{accepted}/count_{required}\right)$$

Proportion quality metric for an observation window:

The proportion quality metric is the ratio of accepted to all observations in the measurement window. In a preferred embodiment, this quality metric is constrained to a value within [0% ... 50%, 100%], where the quality metric is pass if the accepted observations are the majority.

$$ratio_{acceptance} = count_{accepted}/(count_{accepted} + count_{rejected})$$

$$qual_{acceptance} = \max\left((50\% < ratio_{acceptance}), ratio_{acceptance}\right)$$

Quality metric satisfactory:

If the conjunction of the two quality metrics is equal to 100%, the breath rate will be stored in the trends device and queued to be uploaded to the service center. In an alternative embodiment, one or more of the quality metrics, rejection criteria, or other data in the stored fiducial analysis events may accompany the upload.

Confidence interval of tidal amplitude for an observation window:

The average amplitude of accepted Fiducial events that were configured to be included for analysis can be calculated. The amplitudes are summed and then normalized to allow conversion to a mean over the observation window. Further, a preferred embodiment calculates the average absolute deviation of the amplitude (AADamplitude), from the magnitudes of accepted events. The standard deviation can be calculated by scaling the ADD. These measures of variance around a central point may be used to identify disordered breathing when the tidal magnitude falls outside the confidence limits. The observation window to establish the confidence interval may be required to have an unbroken span of accepted analytical fiducial events to establish the reference (FIG. 8).

$$amplitude_{mean} = \left(\sum |Amplitudes_{ax}|\right)\Big/count_{accepted}$$

$$AAD_{amplitude} = \left(\sum |Amplitudes_{ax}| - amplitude_{mean}\right)\Big/count_{accepted}$$

$$STD_{amplitude} = \sqrt{\pi/2} \cdot AAD_{amplitude}$$

In view of the foregoing, the embodiments of the present disclosure provide one or more of the following advantages:

- The ability to resolve morphological features that provide the clinician with measures beyond just event rate, such as tidal ventilation, minute ventilation, inspiration-expiration (IE) ratio, and the like.
- The embodiments of the present disclosure are capable of multiple strategies to optimize the tradeoff space time critical low power measurements versus extended measurement times for more robust estimate of physiologic measure by the selection of a periodic or triggered start, early selection of priority sensors or assessment of all sensors and selection by prospect algorithm, measurement standby, suspend/resume, or deferral, and the like.
- The embodiments of the present disclosure increase the accuracy and statistical power of the results by overlapping analytical fiducial events, rejecting events based on morphological criteria, and quality metrics. No tuning of parameters is required.
- The embodiments of the present disclosure provide a beneficial approach of selecting the most prominent and most recent extrema to prevent false peaks and troughs, that is, by updating a trailing limit with the highest magnitude found for each analysis interval.
- The embodiments of the present disclosure are not as computationally intensive as spectral/frequency analysis approaches.
- The embodiments of the present disclosure are robust to non-stationarity or autonomic variance in the physiologic process.
- The embodiments of the present disclosure are able to resolve event by event (instantaneous) changes in the physiologic process.
- The embodiments of the present disclosure can be applied to physiologic detections from an IMD other than respiration, like oxygen saturation changes, or blood pressure changes from on board sensors.

While the foregoing is directed to embodiments of the disclosure, other and further embodiments of the disclosure may be devised without departing from the scope thereof, and the scope thereof is determined by the claims that follow.

**Claims**

1. A system for analyzing respiratory physiologic signals, comprising:

   - an input configured to receive a discrete-time respiratory physiologic signal; and
   - at least one processor module configured to:

     - compare a sign associated with a present sample value of the respiratory physiologic signal to a sign associated with a previous sample value of the respiratory physiologic signal;
     - determine a presence of a fiducial event if the sign associated with the present sample value is different from the sign associated with the previous sample value;
     - count samples of the respiratory physiologic signal in intervals defined between fiducial events to obtain a respective sample count for each of the intervals; and
     - determine at least one respiratory physiologic parameter based on the sample counts;
     wherein the at least one processor module is configured to reject one or more fiducial events if one or more rejection criteria are met; and
     wherein the one or more rejection criteria relate to an operating limit of at least one signal processing element of the system.

2. The system of claim 1, wherein the respiratory physiologic signal is selected from the group consisting of an electrocardiogram, oxygen saturation, blood pressure, impedance, activity, accelerometry, fluid status, inspiration and/or expiration efforts, and respiration.

3. The system of claim 1 or 2, wherein the fiducial event is a zero-crossing or an extremum of the respiratory physiologic signal.

4. The system of any one of claims 1 to 3, wherein the fiducial event includes, or is, a zero-crossing, and wherein the at least one processor module is configured to identify the zero-crossing if the sign associated with the present sample value differs from the sign associated with the previous sample value.

5. The system of claim 4, wherein the sample count of an interval between two successive zero-crossings corresponds to counts of an unbroken run of a positive sign or a negative sign of the sample values in the interval.

6. The system of any one of claims 1 to 5, wherein the fiducial event includes, or is, an extremum, and wherein the at least one processor module is configured to identify the extremum if the sign associated with a slope the present sample value differs from the sign associated with a slope of the previous sample value.

7. The system of claim 6, wherein the extremum is a peak or a trough.

8. The system of claim 6 or 7, wherein the intervals for the sample count are defined between two successive extrema of the same type.

9. The system of any one of claims 1 to 8, wherein the one or more rejection criteria relate to at least one of a patient's motion, non-monotonicity, and noise.

10. The system of any one of claims 1 to 9, wherein the system is configured to determine the at least one respiratory physiologic parameter using sample counts associated with two or more fiducial events of different types.

11. The system of any one of claims 1 to 10, wherein the system is configured to determine the at least one respiratory physiologic parameter for one or more observation windows, wherein a length of each observation window is based on at least one of a set minimum number of samples and a maximum processing time.

12. The system of any one of claims 1 to 11, further including an implantable medical device, wherein the implantable medical device is configured to perform at least the aspects of comparing signs, determining a presence of a fiducial event, and count samples.

13. A computer implemented method for analyzing respiratory physiologic signals, comprising

- receiving a discrete-time respiratory physiologic signal;
- comparing a sign associated with a present sample value of the respiratory physiologic signal to a sign associated with a previous sample value of the respiratory physiologic signal;
- determining a presence of a fiducial event if the sign associated with the present sample value is different from the sign associated with the previous sample value;
- counting samples of the respiratory physiologic signal in intervals defined between fiducial events to obtain a respective sample count for each of the intervals; and
- determining at least one respiratory physiologic parameter based on the sample counts;

wherein the method further comprises:

- rejecting one or more fiducial events if one or more rejection criteria are met, wherein the one or more rejection criteria relate to an operating limit of at least one signal processing element of the system.

14. A machine-readable storage medium having computer-executable instructions stored, that, when executed, cause one or more processors to perform the method of claim 13.

**Patentansprüche**

1. System zum Analysieren von respiratorischen physiologischen Signalen, umfassend:

   - einen Eingang, der so konfiguriert ist, dass er ein zeitdiskretes respiratorisches physiologisches Signal empfängt; und
   - mindestens ein Prozessormodul, das konfiguriert ist zum:

     - Vergleichen eines Vorzeichens, das mit einem aktuellen Abtastwert des respiratorischen physiologischen Signals verbunden ist, mit einem Vorzeichen, das mit einem vorherigen Abtastwert des respiratorischen physiologischen Signals verbunden ist;
     - Bestimmen des Vorhandenseins eines Referenzereignisses, wenn sich das mit dem aktuellen Abtastwert verbundene Vorzeichen von dem mit dem vorherigen Abtastwert verbundenen Vorzeichen unterscheidet;
     - Zählen von Abtastungen des respiratorischen physiologischen Signals in Intervallen, die zwischen Referenzereignissen definiert sind, um eine entsprechende Abtastanzahl für jedes der Intervalle zu erhalten; und
     - Bestimmen mindestens eines respiratorischen physiologischen Parameters basierend auf der Anzahl der Abtastungen;
     wobei das mindestens eine Prozessormodul so konfiguriert ist, dass es ein oder mehrere Referenzereignisse zurückweist, wenn ein oder mehrere Zurückweisungskriterien erfüllt sind; und
     wobei sich das eine oder die mehreren Zurückweisungskriterien auf eine Betriebsgrenze von mindestens einem Signalverarbeitungselement des Systems beziehen.

2. System nach Anspruch 1, wobei das respiratorische physiologische Signal aus der Gruppe ausgewählt ist, die aus einem Elektrokardiogramm, der Sauerstoffsättigung, dem Blutdruck, der Impedanz, der Aktivität, der Beschleunigungsmessung, dem Flüssigkeitsstatus, den Ein- und/oder Ausatmungsbemühungen und der Respiration besteht.

3. System nach Anspruch 1 oder 2, wobei das Referenzereignis ein Nulldurchgang oder ein Extremum des respiratorischen physiologischen Signals ist.

4. System nach einem der Ansprüche 1 bis 3, wobei das Referenzereignis einen Nulldurchgang einschließt oder ein solcher ist, und wobei das mindestens eine Prozessormodul so konfiguriert ist, dass es den Nulldurchgang identifiziert, wenn sich das mit dem aktuellen Abtastwert verbundene Vorzeichen, von dem mit dem vorherigen Abtastwert verbundenen Vorzeichen unterscheidet.

5. System nach Anspruch 4, wobei die Abtastanzahl eines Intervalls zwischen zwei aufeinanderfolgenden Nulldurchgängen der Anzahl eines ununterbrochenen Laufs eines positiven Vorzeichens oder eines negativen Vorzeichens der Abtastwerte in dem Intervall entspricht.

6. System nach einem der Ansprüche 1 bis 5, wobei das Referenzereignis ein Extremum enthält oder ein solches ist und wobei das mindestens eine Prozessormodul so konfiguriert ist, dass es das Extremum identifiziert, wenn das Vorzeichen, das mit einer Steigung des aktuellen Abtastwerts verbunden ist, sich von dem Vorzeichen unterscheidet, das mit einer Steigung des vorherigen Abtastwerts verbunden ist.

7. System nach Anspruch 6, wobei das Extremum eine Spitze oder ein Tiefpunkt ist.

8. System nach Anspruch 6 oder 7, wobei die Intervalle für die Anzahl von Abtastungen zwischen zwei aufeinanderfolgenden Extrema desselben Typs definiert sind.

9. System nach einem der Ansprüche 1 bis 8, wobei sich das eine oder die mehreren Zurückweisungskriterien auf mindestens eines der Kriterien Bewegung des Patienten, Nicht-Monotonie und Rauschen beziehen.

10. System nach einem der Ansprüche 1 bis 9, wobei das System so konfiguriert ist, dass es den mindestens einen respiratorisch physiologischen Parameter unter Verwendung von Abtastanzahlen bestimmt, die mit zwei oder mehr Referenzereignissen unterschiedlichen Typs verbunden sind.

11. System nach einem der Ansprüche 1 bis 10, wobei das System so konfiguriert ist, dass es den mindestens einen respiratorisch physiologischen Parameter für ein oder mehrere Beobachtungsfenster bestimmt, wobei die Länge jedes Beobachtungsfensters auf einer festgelegten Mindestanzahl von Abtastungen und/oder einer maximalen Verarbeitungszeit basiert.

12. System nach einem der Ansprüche 1 bis 11, das außerdem ein implantierbares medizinisches Gerät enthält, wobei das implantierbare medizinische Gerät so konfiguriert ist, dass es zumindest die Aspekte des Vergleichs von Vorzeichen, des Bestimmens des Vorhandenseins eines Referenzereignisses und des Zählens von Abtastungen durchführt.

13. Computerimplementiertes Verfahren zum Analysieren von respiratorischen physiologischen Signalen, das Folgendes umfasst:

- Empfangen eines zeitdiskreten respiratorischen physiologisches Signals;
- Vergleichen eines Vorzeichens, das mit einem aktuellen Abtastwert des respiratorischen physiologischen Signals verbunden ist, mit einem Vorzeichen, das mit einem vorherigen Abtastwert des respiratorischen physiologischen Signals verbunden ist;
- Bestimmen des Vorhandenseins eines Referenzereignisses, wenn das Vorzeichen, das mit dem aktuellen Abtastwert verbunden ist, sich von dem Vorzeichen unterscheidet, das mit dem vorherigen Abtastwert verbunden ist;
- Zählen von Abtastungen des respiratorischen physiologischen Signals in Intervallen, die zwischen Referenzereignissen definiert sind, um einen jeweiligen Abtastanzahl für jedes der Intervalle zu erhalten; und
- Bestimmen mindestens eines respiratorischen physiologischen Parameters basierend auf der Anzahl der Abtastungen;

wobei das Verfahren ferner umfasst:

- Zurückweisen eines oder mehrerer Referenzereignisse, wenn ein oder mehrere Zurückweisungskriterien erfüllt sind, wobei sich das eine oder die mehreren Zurückweisungskriterien auf eine Betriebsgrenze von mindestens einem Signalverarbeitungselement des Systems beziehen.

14. Maschinenlesbares Speichermedium, auf dem computerausführbare Anweisungen gespeichert sind, die, wenn sie ausgeführt werden, einen oder mehrere Prozessoren veranlassen, das Verfahren nach Anspruch 13 durchzuführen.

**Revendications**

1. Système d'analyse de signaux physiologiques respiratoires, comprenant :

- une entrée configurée pour recevoir un signal physiologique respiratoire de temps discret ; et
- au moins un module de processeur configuré pour :

- comparer un signe associé à une valeur d'échantillon présente du signal physiologique respiratoire à un signe associé à une valeur d'échantillon précédente du signal physiologique respiratoire ;
- déterminer une présence d'un événement repère si le signe associé à la valeur d'échantillon présente est différent du signe associé à la valeur d'échantillon précédente ;
- compter les échantillons du signal physiologique respiratoire dans des intervalles définis entre des événements repères pour obtenir un décompte d'échantillons respectif pour chacun des intervalles ; et
- déterminer au moins un paramètre physiologique respiratoire en se basant sur les décomptes d'échantillons ;
dans lequel l'au moins un module de processeur est configuré pour rejeter un ou plusieurs événements repères si un ou plusieurs critères de rejet sont remplis ; et
dans lequel le ou les critères de rejet concernent une limite de fonctionnement d'au moins un élément de traitement de signal du système.

2. Système selon la revendication 1, dans lequel le signal physiologique respiratoire est choisi dans le groupe constitué par un électrocardiogramme, une saturation en oxygène, une pression sanguine, une impédance, une activité, une accélérométrie, un statut fluidique, des efforts d'inspiration et/ou d'expiration, et une respiration.

3. Système selon la revendication 1 ou 2, dans lequel l'événement repère est un passage par zéro ou un extrémum du signal physiologique respiratoire.

4. Système selon l'une quelconque des revendications 1 à 3, dans lequel l'événement repère inclut, ou est, un passage par zéro, et dans lequel l'au moins un module de processeur est configuré pour identifier le passage par zéro si le signe associé à la valeur d'échantillon présente diffère du signe associé à la valeur d'échantillon précédente.

5. Système selon la revendication 4, dans lequel le décompte d'échantillons d'un intervalle entre deux passages par zéro successifs correspond à des décomptes d'une suite ininterrompue de signes positifs ou de signes négatifs des valeurs d'échantillons dans l'intervalle.

6. Système selon l'une quelconque des revendications 1 à 5, dans lequel l'événement repère inclut, ou est, un extrémum, et dans lequel l'au moins un module de processeur est configuré pour identifier l'extrémum si le signe associé à une pente de la valeur d'échantillon présente diffère du signe associé à une pente de la valeur d'échantillon précédente.

7. Système selon la revendication 6, dans lequel l'extrémum est un pic ou une vallée.

8. Système selon la revendication 6 ou 7, dans lequel les intervalles pour le décompte d'échantillons sont définis entre deux extrémums successifs du même type.

9. Système selon l'une quelconque des revendications 1 à 8, dans lequel le ou les critères de rejet concernent au moins un parmi un mouvement du patient, l'absence de monotonicité et le bruit.

10. Système selon l'une quelconque des revendications 1 à 9, dans lequel le système est configuré pour déterminer l'au moins un paramètre physiologique respiratoire en utilisant des décomptes d'échantillons associés à deux événements repères ou plus de types différents.

11. Système selon l'une quelconque des revendications 1 à 10, dans lequel le système est configuré pour déterminer l'au moins un paramètre physiologique respiratoire pour une ou plusieurs fenêtres d'observation, dans lequel une longueur de chaque fenêtre d'observation est basée sur au moins un parmi un nombre minimal défini d'échantillons et un temps de traitement maximal.

12. Système selon l'une quelconque des revendications 1 à 11, incluant en outre un dispositif médical implantable, dans lequel le dispositif médical implantable est configuré pour réaliser au moins les aspects de comparaison de signes, de détermination d'une présence d'un événement repère, et de décompte des échantillons.

13. Procédé mis en œuvre par ordinateur d'analyse de signaux physiologiques respiratoires, comprenant les étapes consistant à :

   - recevoir un signal physiologique respiratoire de temps discret ;
   - comparer un signe associé à une valeur d'échantillon présente du signal physiologique respiratoire à un signe associé à une valeur d'échantillon précédente du signal physiologique respiratoire ;
   - déterminer une présence d'un événement repère si le signe associé à la valeur d'échantillon présente est différent du signe associé à la valeur d'échantillon précédente ;
   - compter les échantillons du signal physiologique respiratoire dans des intervalles définis entre des événements repères pour obtenir un décompte d'échantillons respectif pour chacun des intervalles ; et
   - déterminer au moins un paramètre physiologique respiratoire en se basant sur les décomptes d'échantillons ;

   dans lequel le procédé comprend en outre les étapes consistant à :

   - rejeter un ou plusieurs événements repères si un ou plusieurs critères de rejet sont remplis, dans lequel le ou les critères de rejet concernent une limite de fonctionnement d'au moins un élément de traitement de signal du système.

14. Support de stockage lisible par machine ayant des instructions exécutables par ordinateur stockées qui, lorsqu'elles sont exécutées, amènent un ou plusieurs processeurs à réaliser le procédé selon la revendication 13.

FIG. 1

EP 4 422 488 B1

FIG. 2

**FIG. 3**

| FIG. 3A | FIG. 3B |
|---------|---------|

Sample IRQ

nxtVal*

nxtVal < 0 — No

Yes

Negative:
nxtSgn = -1

motnFig? — No

Yes

nxtNegMotnRjc = 1

railFig? — No

Yes

nxtNegRailRjc = 1

prmThr < [nxtVal] — No

Yes

nxtNegRjc = 0

lstVal < 0 — No

Yes

Negative:

xngFid = 0
xngDur = 0

xngPrmRjc = 0
xngRailRjc = 0
xngMotnRjc = 0

negCnt + +

Zero-Crossing Down:
    xngFid* = -1
    xngDur* = negCnt + posCnt

Rejection Criteria:
    xngPrmRjc* = lstNegRjc | | nxtPosRjc
    xngRailRjc* = lstNegRailRjc | | nxtPosRailRjc
    xngMotnRjc* = lstNegMotnRjc | | nxtPosMotnRjc

Store*

Save for next extrema:
    xtrPrmRjc* = xngPrmRjc
    xtrRailRjc* = xngRailRjc
    xtrMotnRjc* = xngMotnRjc

Save for trailing lobe:
    lstPosRjc = nxtPosRjc
    lstPosRailRjc = nxtPosRailRjc
    lstPosMotnRjc = nxtPosMotnRjc

Reset for next lobe:
    nxtNegRjc = 1
    nxtNegRailRjc = 0
    nxtNegMotnRjc = 0

Reset negative sample count:
    negCnt = 1

**FIG. 3A**

**Positive:**
nxtSgn = 1

motnFig? — No / Yes

nxtPosMotnRjc = 1

railFig? — No / Yes

nxtPosRailRjc = 1

prmThr < [nxtVal] — No / Yes

nxtPosRjc = 0

lstVal < 0 — Yes / No

**Positive:**
xngFid = 0
xngDur = 0

xngPrmRjc = 0
xngRailRjc = 0
xngMotnRjc = 0

posCnt++

**Zero-Crossing Up:**
xngFid* = 1
xngDur* = posCnt + negCnt

**Rejection Criteria:**
xngPrmRjc* = lstPosRjc || nxtNegRjc
xngRailRjc* = lstPosRailRjc || nxtNegRailRjc
xngMotnRjc* = lstPosMotnRjc || nxtNegMotnRjc

**Store***

**Save for next extrema:**
xtrPrmRjc = xngPrmRjc
xtrRailRjc = xngRailRjc
xtrMotnRjc = xngMotnRjc

**Save for trailing lobe:**
lstNegRjc = nxtNegRjc
lstNegRailRjc = nxtNegRailRjc
lstNegMotnRjc = nxtNegMotnRjc

**Reset for next lobe:**
nxtPosRjc = 1
nxtPosRailRjc = 0
nxtPosMotnRjc = 0

**Reset positive sample count:**
posCnt = 1

xtrDur

**FIG. 3B**

**Input Waveform**

**Next / Last Slope**

**Extreme Trough / Peak**

FIG. 4

FIG. 5

# FIG. 6

| FIG. 6A | FIG. 6C |
|---------|---------|
| FIG. 6B | FIG. 6D |

xtrDur

nxtSgn=-1 — No

Yes

Negative:
nxtDsc++

No — lstVal<nxtVal?

Yes

nxtDscRjc=1

nxtVal<lstVal? — No

Yes

Down:
nxtSlp=-1

Plateau:
nxtSlp=0
nxtAsc++

No — nxtVal<ndrVal
OR xngFid=-1

Yes

nxtAscRjc=1

New Nad ir:
ndrVal*=nxtVal
lstDsc=nxtDsc
lstDscRjc=nxtDscRjc
nxtAscRjc=0
nxtAsc=0

## FIG. 6A

FIG. 6B

Positive:
nxtAsc++

nxtVal<lstVal?
No
Yes

nxtAscRjc=1

lstVal<nxtVal?
No
Yes

Up:
nxtSlp=1

Plateau:
nxtSlp=0
nxtDsc++

apxVal<nxtVal
OR xngFid=1
No
Yes

nxtDscRjc=1

New Peak:
apxVal*=nxtVal
lstAsc=nxtAsc
lstAscRjc=nxtAscRjc
nxtDscRjc=0
nxtDsc=0

## FIG. 6C

```
                    │
                    ▼
              ╱ 0 < lstSlp  ╲
   No        ╱  AND nxtSlp < 1 ╲
 ◄─────────◄                    ►
           ╲                   ╱
            ╲                 ╱
                    │ Yes
                    ▼
┌──────────┐    ┌────────────────────────────────┐
│ prmFid=0 │    │ Peak:                          │
│ PrmDur=0 │    │   prmFid* = 2                  │
└──────────┘    │   prmDur* = lstDsc + lstAsc    │
     │          │   xtrSlpRjc* = lstDscRjc||lstAscRjc │
     │          └────────────────────────────────┘
     │                    │
     │                    ▼
     │              ╱LastprmRD=2╲    No
     │             ◄             ►──────────┐
     │              ╲           ╱           │
     │                    │ Yes            │
     │                    ▼                │
     │         No   ╱ LastapxVal ╲         │
     │      ◄──────◄   < apxVal   ►        │
     │      │       ╲            ╱         │
     │      │            │ Yes             │
     │      │            ▼                 ▼
     │      │   ┌───────────────┐   ┌───────────┐
     │      └──►│ Overwrite*last │   │  Store*   │
     │          └───────────────┘   └───────────┘
     │                 │                  │
     │                 ▼◄─────────────────┘
     │          ┌──────────────────────────┐
     │          │ Save for next zero-crossing: │
     │          │  xngSlpRjc* = xtrSlpRjc  │
     │          └──────────────────────────┘
     │                      │
     │                      ▼
     │              ╭──────────────╮
     └─────────────►│    xtrLst    │
                    ╰──────────────╯
```

**FIG. 6D**

strLst

Save for next sample:
lstVal=nxtVal
lstSgn=nxtSgn
lstSlp=nxtSlp

Time<Observation Limit
AND Count<Event Limit

Yes → End IRQ

No

Disable Sample IRQ

Observation Complete

FIG. 7

FIG. 8

Rest Period Start

Heart Rate Motion Flag

Rest<HR OR Motion Flag? — No → Select sensor subset

Yes

Standby? — Yes

No

Rest Period End <Now+Wait — No → Wait 15 minutes

Yes

Free Resources
Wait for next Rest Period

End

Set observation window limit:
- Time limit
- Event cout limit

Enable Sample IRQ

Observation Complete

Post-Observation Window Analysis:
Respiration per Minute
Sufficient Observations Quality Metric
Proportion Quality Metric

Quality Metrics
Saticfactory

Yes

Store Results in Trends
Upload to Service Center

Free Resources
Wait for next Rest Period

End

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5300093 A

- US 2016279361 A